# EUROPEAN PATENT APPLICATION

(11) **EP 4 631 936 A1**
(43) Date of publication of application: **15.10.2025**
(21) Application number: 25166047.8
(22) Date of filing: 25.03.2025
(51) Int. Cl.: C07D 303/04, C07C 49/17, C07C 67/08, C07C 69/16, C07D 317/26, C07F 3/02

(54) **5-(2-BUTYL-1,3-DIOXOLAN-2-YL)-1-METHYLPENTYL ACETATE AND PROCESS FOR PREPARING 2,7-DIACETOXYUNDECANE THEREFROM**

(30) Priority: 09.04.2024 JP 2024062647
(71) Applicant: SHIN-ETSU CHEMICAL CO., LTD., Tokyo 1000005 (JP)
(72) Inventor: MIYAKE, Yuki, Niigata, 942-8601 (JP); WATANABE, Takeru, Niigata, 942-8601 (JP); NAGAE, Yusuke, Niigata, 942-8601 (JP)
(74) Representative: De Vries & Metman

(57) **Abstract**

The present invention provides 5-(2-butyl-1,3-dioxolan-2-yl)-1-methylpentyl acetate of the following formula (1).

The present invention further provides the aforesaid 5-(2-butyl-1,3-dioxolan-2-yl)-1-methylpentyl acetate (1), the process comprising the step of subjecting an organic magnesium compound (2) of the following general formula (2), wherein X¹ represents a halogen atom or a 3-(2-butyl-1,3-dioxolan-2-yl)propyl group, to a nucleophilic addition reaction with propylene oxide of the following formula (3) and then reacting with an acetylating agent of the following general formula (4), wherein X² represents a halogen atom, an acetoxy group, a methoxy group, or an ethoxy group, to form 5-(2-butyl-1,3-dioxolan-2-yl)-1-methylpentyl acetate (1).

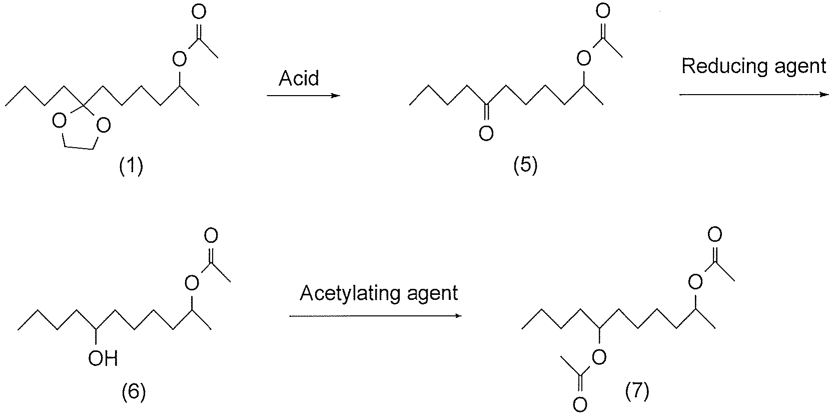

## Description

### TECHNICAL FIELD

The present invention relates to 5-(2-butyl-1,3-dioxolan-2-yl)-1-methylpentyl acetate and a process for preparing 2,7-diacetoxyundecane therefrom.

### BACKGROUND ART

The Pear gall midge (scientific name: *Contarinia pyrivora*) is a European pest against fruit, such as pears. Pear gall midge larvae penetrate and feed on the fruit, causing the fruit to turn black and fall from the tree. Control is difficult because the larvae penetrate the fruit, often making typical pesticides ineffective due to poor contact. Biological control methods are attracting attention due to concerns about residual pesticides, and one promising method is the use of sex pheromones (Non-Patent Literature 1 below).

A sex pheromone composition of the Pear gall midge is reported to be a mixture of 2,7-diacetoxyundecane and 7-(acetoxy)-2-undecanone (Non-Patent Literature 2 below).

A process for preparing the major component, 2,7-diacetoxyundecane, of the aforesaid mixture is reported in Non-Patent Literature 2 below, and includes, for example, reacting 1,4-dibromobutane, 1-pentanal, and acetaldehyde with magnesium in tetrahydrofuran (THF) to synthesize a mixture of 5,10-tetradecanediol, 2,7-octanediol, and 2,7-undecanediol. The mixture of 5,10-tetradecanediol, 2,7-octanediol, and 2,7-undecanediol thus obtained is then separated by silica gel column chromatography to isolate and purify 2,7-undecanediol. 2,7-Undecanediol thus obtained is then acetylated with acetic anhydride in the presence of pyridine to obtain 2,7-diacetoxyundecane.

### PRIOR ART

### [Non-Patent Literatures]

[Non-Patent Literature 1] Elisabeth A. Hodgdon et al., The Canadian Entomologist, 2022, 154, e37, 1-17.
[Non-Patent Literature 2] Lakmali Amarawardana et al., Thesis Uni. Greenwich, 2009, 1-184.

### OBJECT OF THE INVENTION

However, the yield of the process for preparing 2,7-diacetoxyundecane according to Non-Patent Literature 2 is estimated to be poor because three types of diols are produced in the first step, and because the intermediate of the target compound, 2,7-undecanediol, is highly water-soluble. Industrialization of the preparation process also is difficult because acetaldehyde used in the aforesaid first step is carcinogenic and causes sick building syndrome, requiring special-purpose equipment for handling as well as strict wastewater treatment. Industrialization of the preparation process also is difficult because the silica gel column chromatography used to separate the three types of diols is difficult to carry out on an industrial scale (of more than 100 kg).

The present invention has been made in view of the aforementioned circumstances, and aims to provide a novel compound as a synthetic intermediate for efficiently preparing 2,7-diacetoxyundecane. The present invention also aims to provide a process for preparing the novel compound, and a process for preparing 2,7-diacetoxyundecane from the novel compound.

### SUMMARY OF THE INVENTION

As a result of intensive research to overcome the aforesaid problems of the prior art, the present inventors found a novel compound, 5-(2-butyl-1,3-dioxolan-2-yl)-1-methylpentyl acetate. The aforesaid 5-(2-butyl-1,3-dioxolan-2-yl)-1-methylpentyl acetate according to the present invention may be prepared inexpensively and in large amounts, and may be purified by only distillation. The present inventors also found that 2,7-diacetoxyundecane may be efficiently prepared from the aforesaid 5-(2-butyl-1,3-dioxolan-2-yl)-1-methylpentyl acetate.

According to a first aspect of the present invention, there is provided 5-(2-butyl-1,3-dioxolan-2-yl)-1-methylpentyl acetate of the following formula (1):

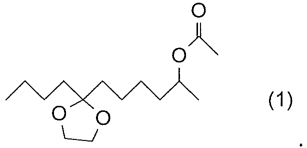

According to a second aspect of the present invention, there is provided a process for preparing the aforesaid 5-(2-butyl-1,3-dioxolan-2-yl)-1-methylpentyl acetate (1), the process comprising the step of:
subjecting an organic magnesium compound (2) of the following general formula (2): 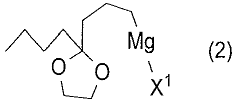
wherein X¹ represents a halogen atom or a 3-(2-butyl-1,3-dioxolan-2-yl)propyl group, to a nucleophilic addition reaction with propylene oxide of the following formula (3): 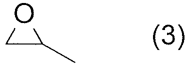
and then reacting with an acetylating agent of the following general formula (4): 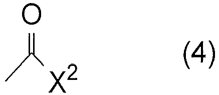
wherein X² represents a halogen atom, an acetoxy group, a methoxy group, or an ethoxy group,
to form 5-(2-butyl-1,3-dioxolan-2-yl)-1-methylpentyl acetate (1).

According to a third aspect of the present invention, there is provided a process for preparing 10-acetoxy-5-undecanone of the following formula (5): 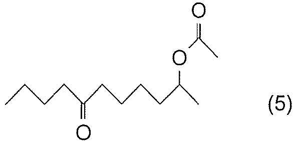
the process comprising the step of:
   eliminating a ketal from 5-(2-butyl-1,3-dioxolan-2-yl)-1-methylpentyl acetate of the following formula (1): 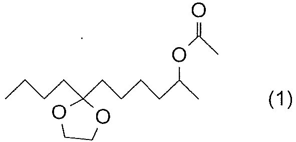
in the presence of an acid or a peroxide to form 10-acetoxy-5-undecanone (5).

According to a fourth aspect of the present invention, there is provided a process for preparing 2,7-diacetoxyundecane of the following formula (7): 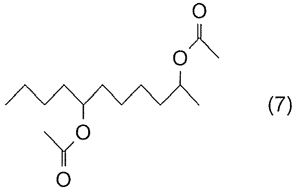 the process comprising the step of:
subjecting the aforesaid 10-acetoxy-5-undecanone (5) to a reduction reaction to form 10-acetoxy-5-undecanol of the following formula (6): 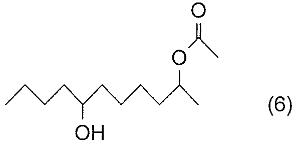 and
subjecting the aforesaid 10-acetoxy-5-undecanol (6) to an acetylation reaction to form 2,7-diacetoxyundecane (7).

According to the present invention, the target compound, 2,7-diacetoxyundecane, may be efficiently prepared with a high yield and a low environmental impact. According to the present invention, the target compound, 2,7-diacetoxyundecane, may be economically prepared. According to the present invention, substantially no compounds that are similar to the target compound are formed in the steps, making purification by column chromatography unnecessary, and allowing purification by distillation, which may be scaled up. According to the present invention, there is also provided a novel synthetic intermediate useful for preparing the aforesaid 2,7-diacetoxyundecane.

### DETAILED DESCRIPTION OF THE INVENTION

### I. Process for preparing 5-(2-butyl-1,3-dioxolan-2-yl)-1-methylpentyl acetate (1) of the following general formula (1)

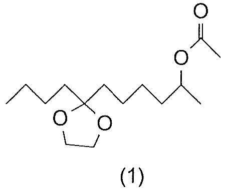

A process for preparing the aforesaid 5-(2-butyl-1,3-dioxolan-2-yl)-1-methylpentyl acetate (1) will be explained in detail below.

5-(2-Butyl-1,3-dioxolan-2-yl)-1-methylpentyl acetate (1) may be prepared, for example, by subjecting an organic magnesium compound of the following general formula (2) to a nucleophilic addition reaction with propylene oxide of the following formula (3), and then subjecting the reaction mixture to acetylation with an acetylating agent of the following general formula (4), as shown in the following chemical reaction formula: 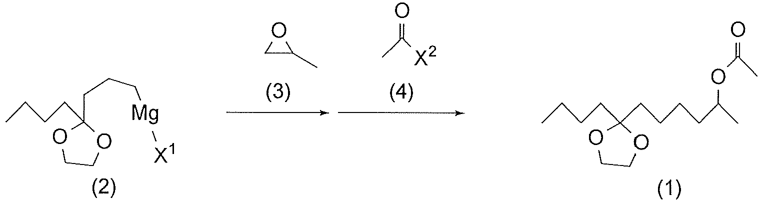
(i) The aforesaid organic magnesium compound (2) will be described in detail below.

In the general formula (2) above, X¹ represents a halogen atom or a 3-(2-butyl-1,3-dioxolan-2-yl)propyl group. Specific examples of the halogen atom X¹ include a chlorine atom, a bromine atom, and an iodine atom. The halogen atom X¹ is preferably a chlorine atom and a bromine atom, and more preferably a chlorine atom. By using said chlorine atom and bromine atom, a preferred ease of handling may be ensured. By using said chlorine atom, a more preferred ease of handling may be ensured.

Specific examples of the organic magnesium compound (2) include organic magnesium compounds (Grignard reagents) such as [3-(2-butyl-1,3-dioxolan-2-yl)propyl]magnesium chloride, [3-(2-butyl-1,3-dioxolan-2-yl)propyl]magnesium bromide, and [3-(2-butyl-1,3-dioxolan-2-yl)propyl]magnesium iodide. The organic magnesium compound (2) is preferably [3-(2-butyl-1,3-dioxolan-2-yl)propyl]magnesium chloride. By using said [3-(2-butyl-1,3-dioxolan-2-yl)propyl]magnesium chloride, a preferred ease of preparation (availability) may be ensured.

The organic magnesium compound (2) may be used alone or in combination thereof, if necessary. The organic magnesium compound (2) also may be prepared, for example, by a preparation process that will be explained below.

(ii) A nucleophilic addition reaction of the organic magnesium compound (2) with propylene oxide (3), followed by acetylation with an acetylating agent (4) will be described in detail below.

In the nucleophilic addition reaction, the amount of propylene oxide (3) used, per mol of the organic magnesium compound (2), is preferably 0.8 to 2.0 mol, more preferably 0.9 to 1.7 mol, and even more preferably 1.0 to 1.5 mol. By using said preferred amount, said more preferred amount, and said even more preferred amount, preferred economy, more preferred economy, and even more preferred economy may be ensured.

A solvent may be incorporated in the nucleophilic addition reaction, if necessary. Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; and polar solvents such as *N,N*-dimethylformamide (DMF), N,N-dimethylacetamide (DMAC), *N*-methylpyrrolidone (NMP), dimethyl sulfoxide (DMSO), γ-butyrolactone (GBL), acetonitrile, *N,N'*-dimethylpropylene urea (DMPU), hexamethylphosphoric triamide (HMPA), dichloromethane, and chloroform. Hydrocarbon solvents such as toluene and xylene; and ether solvents such as tetrahydrofuran, 2-methyltetrahydrofuran, and 4-methyltetrahydropyran are preferred. By using said hydrocarbon solvents such as toluene and xylene; and ether solvents such as tetrahydrofuran, 2-methyltetrahydrofuran, and 4-methyltetrahydropyran, a preferred reactivity may be ensured. Tetrahydrofuran, 2-methyltetrahydrofuran, and 4-methyltetrahydropyran are more preferred. By using said tetrahydrofuran, 2-methyltetrahydrofuran, and 4-methyltetrahydropyran, a more preferred reactivity may be ensured.

The solvent may be used alone or in combination thereof, if necessary. The solvent may be a commercially available one.

The amount of the solvent used, per mol of the organic magnesium compound (2), is preferably 30 to 5,000 g, more preferably 50 to 3,000 g, and even more preferably 100 to 1,000 g. By using said preferred amount, said more preferred amount, and said even more preferred amount, a preferred reactivity, a more preferred reactivity, and an even more preferred reactivity may be ensured.

The nucleophilic addition reaction may be carried out in the presence of a catalyst, if necessary.

Examples of the catalyst include copper compounds such as cuprous halides such as cuprous chloride, cuprous bromide, and cuprous iodide; and cupric halides such as cupric chloride, cupric bromide, and cupric iodide. Cuprous halides are preferred. By using said cuprous halides, a preferred reactivity may be ensured. Cuprous chloride is more preferred. By using said cuprous chloride, a more preferred reactivity may be ensured.

The catalyst may be used alone or in combination thereof, if necessary. The catalyst may be a commercially available one.

The amount of the catalyst used, per mol of the organic magnesium compound (2), is preferably 0.0003 to 0.3 mol, and more preferably 0.001 to 0.1 mol. By using said preferred amount and said more preferred amount, a preferred reaction rate and/or post-treatment and a more preferred reaction rate and/or post-treatment may be ensured.

The reaction temperature of the nucleophilic addition reaction varies, depending on the organic magnesium compound (2) used, and is preferably -78 to 70°C, more preferably -20 to 50°C, and even more preferably 0 to 30°C. By using said preferred reaction temperature, said more preferred reaction temperature, and said even more preferred reaction temperature, a preferred reactivity, a more preferred reactivity, and an even more preferred reactivity may be ensured.

The reaction time of the nucleophilic addition reaction varies, depending on the solvent to be used and/or the production scale, and is preferably 0.5 to 100 hours. By using said preferred reaction time, a preferred reactivity may be ensured.

Examples of the acetylating agent used in the acetylation reaction include acetyl halides such as acetyl chloride, acetyl bromide, and acetyl iodide; acetic anhydride; and alkyl acetates such as methyl acetate and ethyl acetate. Ethyl acetate, acetyl chloride, and acetic anhydride are preferred. By using said ethyl acetate, acetyl chloride, and acetic anhydride, a preferred reactivity may be ensured. Acetyl chloride and acetic anhydride are more preferred. By using said acetyl chloride and acetic anhydride, a more preferred reactivity may be ensured. Acetic anhydride is even more preferred. By using said acetic anhydride, an even more preferred reactivity may be ensured.

In the acetylation reaction, the amount of the acetylating agent (4) used, per mol of the organic magnesium compound (2), is preferably 0.8 to 2.0 mol, more preferably 0.9 to 1.7 mol, and even more preferably 1.0 to 1.5 mol. By using said preferred amount, said more preferred amount, and said even more preferred amount, preferred economy, more preferred economy, and even more preferred economy may be ensured.

A solvent may be incorporated in the acetylation reaction, if necessary. Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; and polar solvents such as N,N-dimethylformamide (DMF), N,N-dimethylacetamide (DMAC), N-methylpyrrolidone (NMP), dimethyl sulfoxide (DMSO), γ-butyrolactone (GBL), acetonitrile, N,N'-dimethylpropylene urea (DMPU), hexamethylphosphoric triamide (HMPA), dichloromethane, and chloroform. Hydrocarbon solvents such as toluene and xylene; and ether solvents such as tetrahydrofuran, 2-methyltetrahydrofuran, and 4-methyltetrahydropyran are preferred. By using said hydrocarbon solvents such as toluene and xylene; and ether solvents such as tetrahydrofuran, 2-methyltetrahydrofuran, and 4-methyltetrahydropyran, a preferred reactivity may be ensured. Tetrahydrofuran, 2-methyltetrahydrofuran, and 4-methyltetrahydropyran are more preferred. By using said tetrahydrofuran, 2-methyltetrahydrofuran, and 4-methyltetrahydropyran, a more preferred reactivity may be ensured.

The solvent may be used alone or in combination thereof, if necessary. The solvent may be a commercially available one.

The amount of the solvent used, per mol of the organic magnesium compound (2), is preferably 30 to 5,000 g, more preferably 50 to 3,000 g, and even more preferably 100 to 1,000 g. By using said preferred amount, said more preferred amount, and said even more preferred amount, a preferred reactivity, a more preferred reactivity, and an even more preferred reactivity may be ensured.

The reaction temperature of the acetylation reaction varies, depending on the organic magnesium compound (2) used, and is preferably -30 to 100°C, more preferably 0 to 80°C, and even more preferably 20 to 60°C. By using said preferred reaction temperature, said more preferred reaction temperature, and said even more preferred reaction temperature, a preferred reactivity, a more preferred reactivity, and an even more preferred reactivity may be ensured. Salts may precipitate and make stirring difficult at low temperatures in the acetylation step, and so the reaction temperature is preferably 20°C or higher. By using said preferred reaction temperature, a preferred stirring efficiency may be ensured.

The reaction time of the acetylation reaction varies, depending on the solvent to be used and/or the production scale, and is preferably 0.5 to 100 hours. By using said preferred reaction time, a preferred reactivity may be ensured.

Although 5-(2-butyl-1,3-dioxolan-2-yl)-1-methylpentyl acetate (1) is prepared by reacting with propylene oxide and then reacting with an acetylating agent, it is also possible to synthesize 10-hydroxy-5-undecanone by reacting with propylene oxide without subsequently reacting with an acetylating agent, and then synthesizing 2,7-diacetoxyundecane via 2,7-undecanediol. However, in such a case, the free hydroxy group may react with ketone intramolecularly/intermolecularly in the step of removing acetal. It is also estimated that extraction after the reaction is more difficult for 2,7-undecanediol than for the intermediate of the present invention, 10-acetoxy-5-undecanol (6), due to the high water solubility of 2,7-undecanediol. Based on such a background, it is desirable to react with propylene oxide, and then react with an acetylating agent to prepare 5-(2-butyl-1,3-dioxolan-2-yl)-1-methylpentyl acetate (1), which is then used as a key intermediate to synthesize 2,7-diacetoxyundecane.

(iii) A process for preparing the aforesaid organic magnesium compound (2) will be described in detail below.

The organic magnesium compound (2) may be prepared according to the following process.

The organic magnesium compound (2) is a Grignard reagent.

The organic magnesium compound (2) may be prepared, for example, by reacting a 2-butyl-2-(3-halopropyl)-1,3-dioxolane compound of the following general formula (8) with magnesium in a solvent at a certain temperature, as shown in the following chemical reaction formula: 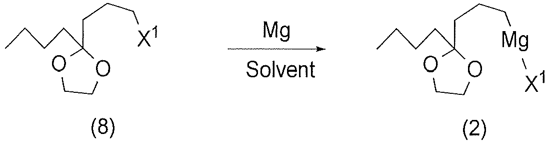
(a) First, the aforesaid 2-butyl-2-(3-halopropyl)-1,3-dioxolane compound (8) will be described in detail below.

In the general formula (8) above, X¹ represents a halogen atom. Specific examples of the halogen atom X¹ include a chlorine atom, a bromine atom, and an iodine atom. The halogen atom X¹ is preferably a bromine atom and a chlorine atom, and more preferably a chlorine atom. By using said bromine atom and chlorine atom, a preferred ease of handling may be ensured. By using said chlorine atom, a more preferred ease of handling may be ensured.

Specific examples of the 2-butyl-2-(3-halopropyl)-1,3-dioxolane compound (8) include 2-butyl-2-(3-chloropropyl)-1,3-dioxolane, 2-butyl-2-(3-bromopropyl)-1,3-dioxolane, and 2-butyl-2-(3-iodopropyl)-1,3-dioxolane.

The 2-butyl-2-(3-halopropyl)-1,3-dioxolane compound (8) may be used alone or in combination thereof, if necessary. The 2-butyl-2-(3-halopropyl)-1,3-dioxolane compound (8) may be a commercially available one or may be synthesized in house.

The amount of magnesium used, per mol of the 2-butyl-2-(3-halopropyl)-1,3-dioxolane compound (8), is preferably 0.8 to 2.0 gram atoms, more preferably 0.9 to 1.6 gram atoms, and even more preferably 1.0 to 1.4 gram atoms. By using said preferred amount, said more preferred amount, and said even more preferred amount, a preferred completion of the reaction, a more preferred completion of the reaction, and an even more preferred completion of the reaction may be ensured.

Examples of the solvent include general solvents such as ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; and hydrocarbon solvents such as hexane, heptane, benzene, toluene, xylene, and cumene. Hydrocarbon solvents such as toluene and xylene; and ether solvents such as tetrahydrofuran, 2-methyltetrahydrofuran, and 4-methyltetrahydropyran are preferred. By using said hydrocarbon solvents such as toluene and xylene; and ether solvents such as tetrahydrofuran, 2-methyltetrahydrofuran, and 4-methyltetrahydropyran, a preferred reaction rate of forming the aforesaid Grignard reagent may be ensured. Tetrahydrofuran, 2-methyltetrahydrofuran, and 4-methyltetrahydropyran are more preferred. By using said tetrahydrofuran, 2-methyltetrahydrofuran, and 4-methyltetrahydropyran, a more preferred reaction rate of forming the aforesaid Grignard reagent may be ensured. Tetrahydrofuran is even more preferred. By using said tetrahydrofuran, an even more preferred reaction rate of forming the aforesaid Grignard reagent may be ensured.

The solvent may be used alone or in combination thereof, if necessary. The solvent may be a commercially available one.

The amount of the solvent used, per mol of the 2-(3-halopropyl)-2-butyl-1,3-dioxolane compound (8), is preferably 30 to 5,000 g, more preferably 50 to 3,000 g, and even more preferably 100 to 1,000 g. By using said preferred amount, said more preferred amount, and said even more preferred amount, a preferred reactivity, a more preferred reactivity, and an even more preferred reactivity may be ensured.

The reaction temperature of the aforesaid reaction with magnesium is preferably -10 to 80°C, more preferably 10 to 70°C, even more preferably 30 to 65°C, and especially preferably 45 to 60°C. By using said preferred reaction temperature, said more preferred reaction temperature, said even more preferred reaction temperature, and said especially preferred reaction temperature, a preferred suppression of side reactions, a more preferred suppression of side reactions, an even more preferred suppression of side reactions, and an especially preferred suppression of side reactions may be ensured.

The reaction time of the aforesaid reaction with magnesium varies, depending on the solvent to be used and/or the production scale, and is preferably 0.5 to 100 hours, more preferably 1 to 30 hours, even more preferably 1.5 to 15 hours, and especially preferably 2 to 8 hours. By using said preferred reaction time, said more preferred reaction time, said even more preferred reaction time, and said especially preferred reaction time, a preferred reactivity, a more preferred reactivity, an even more preferred reactivity, and an especially preferred reactivity may be ensured.

A Grignard reagent typically is prepared by reacting a corresponding halide with magnesium. However, the aforesaid Grignard reagent, organic magnesium compound (2), proceeds via a cyclization reaction above a certain temperature. Specifically, the cyclization reaction proceeds gradually above an internal temperature of 60°C, and cyclization proceeds quickly above 95°C to form a four-membered ring compound. The temperature is therefore extremely important to produce the target Grignard reagent, organic magnesium compound (2), with high yield, and preparation at 30 to 60°C is desirable.

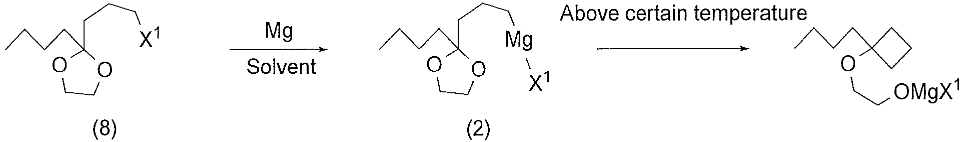

(b) A process for preparing the aforesaid 2-butyl-2-(3-halopropyl)-1,3-dioxolane compound (8) will be described in detail below.

The 2-butyl-2-(3-halopropyl)-1,3-dioxolane compound (8) may be prepared in a conventional method or a process described below.

The 2-butyl-2-(3-halopropyl)-1,3-dioxolane compound (8) may be prepared, for example, by subjecting a 1-halo-4-octanone compound of the following general formula (9) to an acetalization reaction with ethylene glycol of the following formula (10) in the presence of an acid, as shown in the following chemical reaction formula:

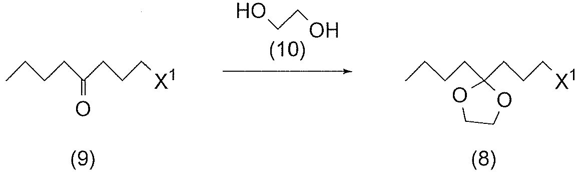

The aforesaid 1-halo-4-octanone compound (9) will be described in detail below.

In the general formula (9) above, X¹ represents a halogen atom. Specific examples of the halogen atom X¹ include a chlorine atom, a bromine atom, and an iodine atom. The halogen atom X¹ is preferably a bromine atom and a chlorine atom.

Specific examples of the 1-halo-4-octanone compound (9) include 1-chloro-4-octanone, 1-bromo-4-octanone, and 1-iodo-4-octanone. 1-Chloro-4-octanone and 1-bromo-4-octanone are preferred. By using said 1-chloro-4-octanone and 1-bromo-4-octanone, a preferred ease of preparation (availability) may be ensured.

The 1-halo-4-octanone compound (9) may be used alone or in combination thereof, if necessary. The 1-halo-4-octanone compound (9) may be a commercially available one or may be prepared, for example, by subjecting 4-chlorobutyryl chloride to a nucleophilic substitution reaction with a butylmagnesium halide.

In the acetalization reaction, the amount of ethylene glycol (10) used, per mol of the 1-halo-4-octanone compound (9), is preferably 0.8 to 2.0 mol, more preferably 0.9 to 1.7 mol, and even more preferably 1.0 to 1.5 mol. By using said preferred amount, said more preferred amount, and said even more preferred amount, preferred economy, more preferred economy, and even more preferred economy may be ensured.

An acid may be incorporated in the acetalization reaction, if necessary.

Examples of the acid include mineral acids such as hydrochloric acid, sulfuric acid, and nitric acid; carboxylic acids such as formic acid and oxalic acid; aromatic sulfonic acids such as benzenesulfonic acid and p-toluenesulfonic acid; and Lewis acids such as aluminum trichloride, aluminum ethoxide, aluminum isopropoxide, aluminum oxide, boron trifluoride, boron trichloride, boron tribromide, magnesium chloride, magnesium bromide, magnesium iodide, zinc chloride, zinc bromide, zinc iodide, tin tetrachloride, tin tetrabromide, dibutyltin dichloride, dibutyltin dimethoxide, dibutyltin oxide, magnesium chloride, magnesium bromide, titanium tetrachloride, titanium tetrabromide, titanium(IV) methoxide, titanium(IV) ethoxide, titanium(IV) isopropoxide, and titanium(IV) oxide. Benzenesulfonic acid and p-toluenesulfonic acid are preferred. By using said benzenesulfonic acid and *p*-toluenesulfonic acid, a preferred reactivity may be ensured.

The acid may be used alone or in combination thereof, if necessary.

When the acid is used, the amount of the acid used, per mol of the 1-halo-4-octanone compound (9), is preferably 0.0001 to 1.00 mol, more preferably 0.0005 to 0.5 mol, and even more preferably 0.001 to 0.1 mol. By using said preferred amount, said more preferred amount, and said even more preferred amount, a preferred reactivity and economy, a more preferred reactivity and economy, and an even more preferred reactivity and economy may be ensured.

Trialkyl orthoformate may be incorporated in the acetalization reaction, if necessary.

Examples of trialkyl orthoformate include trimethyl orthoformate and triethyl orthoformate. Trimethyl orthoformate is preferred.

When the trialkyl orthoformate is used, the amount of the trialkyl orthoformate used, per mol of the 1-halo-4-octanone compound (9), is preferably 0.8 to 2.00 mol, more preferably 0.9 to 1.7 mol, and even more preferably 1.0 to 1.5 mol. By using said preferred amount, said more preferred amount, and said even more preferred amount, a preferred reactivity and economy, a more preferred reactivity and economy, and an even more preferred reactivity and economy may be ensured.

A solvent may be incorporated in the acetalization reaction, if necessary. Examples of the solvent include general solvents such as ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; and polar solvents such as N,N-dimethylformamide (DMF), N,N-dimethylacetamide (DMAC), *N*-methylpyrrolidone (NMP), dimethyl sulfoxide (DMSO), γ-butyrolactone (GBL), acetonitrile, *N,N'*-dimethylpropylene urea (DMPU), hexamethylphosphoric triamide (HMPA), dichloromethane, and chloroform. Hydrocarbon solvents such as toluene and xylene; and ether solvents such as tetrahydrofuran, 2-methyltetrahydrofuran, and 4-methyltetrahydropyran are preferred. By using said hydrocarbon solvents such as toluene and xylene; and ether solvents such as tetrahydrofuran, 2-methyltetrahydrofuran, and 4-methyltetrahydropyran, a preferred reactivity may be ensured. Hexane, toluene, and xylene are more preferred. By using said hexane, toluene, and xylene, a more preferred reactivity may be ensured. Toluene and xylene are even more preferred. By using said toluene and xylene, an even more preferred reactivity may be ensured.

The solvent may be used alone or in combination thereof, if necessary. The solvent may be a commercially available one.

The amount of the solvent used, per mol of the 1-halo-4-octanone compound (9), is preferably 30 to 5,000 g, more preferably 50 to 3,000 g, and even more preferably 100 to 1,000 g. By using said preferred amount, said more preferred amount, and said even more preferred amount, a preferred reactivity, a more preferred reactivity, and an even more preferred reactivity may be ensured.

The reaction temperature of the acetalization reaction is preferably 0 to 120°C, more preferably 10 to 100°C, and even more preferably 40 to 80°C. By using said preferred reaction temperature, said more preferred reaction temperature, and said even more preferred reaction temperature, a preferred reactivity, a more preferred reactivity, and an even more preferred reactivity may be ensured.

The reaction time of the acetalization reaction varies, depending on the solvent to be used and/or the production scale, and is preferably 0.5 to 100 hours. By using said preferred reaction time, a preferred reactivity may be ensured.

### II. Process for preparing 2,7-diacetoxyundecane of the following general formula (7)

One target compound of the present invention, 2,7-diacetoxyundecane of the following general formula (7), is prepared according to the preparation process of the following chemical reaction formula. Specifically, acetal of 5-(2-butyl-1,3-dioxolan-2-yl)-1-methylpentyl acetate (1) is eliminated to form 10-acetoxy-5-undecanone (5). Only the ketone site of the aforesaid 10-acetoxy-5-undecanone (5) is then selectively reduced with a reducing agent to form 10-acetoxy-5-undecanol (6). The hydroxy group of the aforesaid 10-acetoxy-5-undecanol (6) is then acetylated with an acetylating agent to form 2,7-diacetoxyundecane (7).

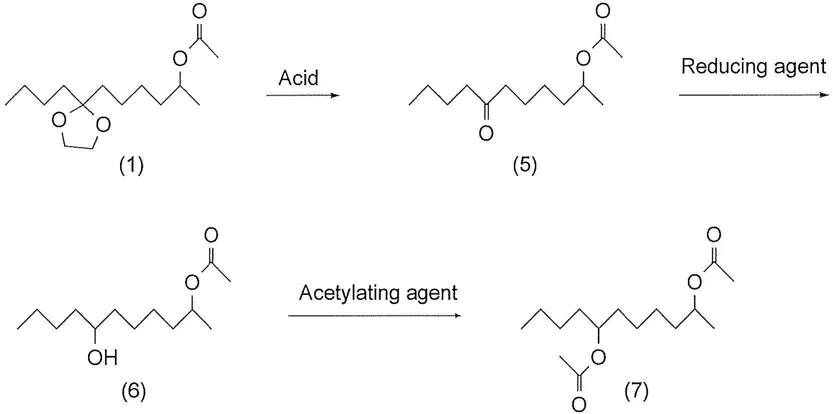

### II. Process for preparing the aforesaid 10-acetoxy-5-undecanone (5)

(i) First, the process for preparing the 10-acetoxy-5-undecanone (5) will be explained in detail below.

10-Acetoxy-5-undecanone (5) may be prepared by eliminating acetal of 5-(2-butyl-1,3-dioxolan-2-yl)-1-methylpentyl acetate (1) with an acid or a peroxide.

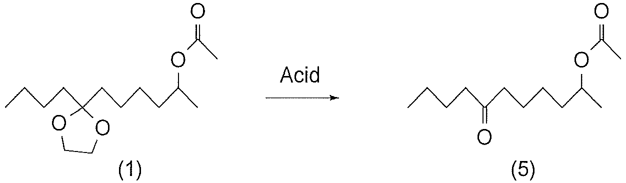

The deprotection may be carried out, for example, in the presence of an acid or a peroxide. Specifically, the deprotection may be carried out, for example, by (1) an exchange reaction with an acid, (2) an acid hydrolysis reaction, or (3) an oxidation reaction with a peroxide.

Examples of the acids of the exchange reaction and acid hydrolysis reaction include mineral acids such as hydrochloric acid, sulfuric acid, and nitric acid; carboxylic acids such as formic acid and acetic acid; aromatic sulfonic acids such as benzenesulfonic acid and *p*-toluenesulfonic acid; and Lewis acids such as aluminum trichloride, aluminum ethoxide, aluminum isopropoxide, aluminum oxide, boron trifluoride, boron trichloride, boron tribromide, magnesium chloride, magnesium bromide, magnesium iodide, zinc chloride, zinc bromide, zinc iodide, tin tetrachloride, tin tetrabromide, dibutyltin dichloride, dibutyltin dimethoxide, dibutyltin oxide, magnesium chloride, magnesium bromide, titanium tetrachloride, titanium tetrabromide, titanium(IV) methoxide, titanium(IV) ethoxide, titanium(IV) isopropoxide, and titanium(IV) oxide. Benzenesulfonic acid and p-toluenesulfonic acid are preferred. By using said benzenesulfonic acid and p-toluenesulfonic acid, a preferred reactivity may be ensured.

The acid may be used alone or in combination thereof, if necessary.

When the acid is used, the amount of the acid used, per mol of 5-(2-butyl-1,3-dioxolan-2-yl)-1-methylpentyl acetate (1), is preferably 0.0001 to 1.00 mol, more preferably 0.0005 to 0.5 mol, and even more preferably 0.001 to 0.1 mol. By using said preferred amount, said more preferred amount, and said even more preferred amount, a preferred reactivity and economy, a more preferred reactivity and economy, and an even more preferred reactivity and economy may be ensured.

Examples of the peroxide in the oxidation reaction include perchloric acid (HClO₄), tert-butylhydroperoxide (*t*-BuOOH), and 2,3-dichloro-5,6-dicyano-1,4-benzoquinone (DDQ).

Water may be incorporated in the deprotection reaction together with the aforementioned acid, if necessary. The amount of the water used, per mol of 5-(2-butyl-1,3-dioxolan-2-yl)-1-methylpentyl acetate (1), is preferably more than 0 up to 7,000 g, more preferably 10 to 3,000 g, and even more preferably 18 to 1,000 g. By using said preferred amount, said more preferred amount, and said even more preferred amount, a preferred reactivity, a more preferred reactivity, and an even more preferred reactivity may be ensured.

A solvent may be incorporated in the deprotection reaction together with the aforementioned acid or water, if necessary.

Examples of the solvent include ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; polar solvents such as *N,N*-dimethylformamide (DMF), *N,N*-dimethylacetamide (DMAC), *N-*methylpyrrolidone (NMP), dimethyl sulfoxide (DMSO), γ-butyrolactone (GBL), acetonitrile, acetone, *N,N'-*dimethylpropylene urea (DMPU), hexamethylphosphoric triamide (HMPA), dichloromethane, and chloroform; ester solvents such as methyl acetate, ethyl acetate, n-propyl acetate, and n-butyl acetate; and alcoholic solvents such as methanol and ethanol. Polar solvents such as *N,N-*dimethylformamide (DMF), *N,N-*dimethylacetamide (DMAC), *N*-methylpyrrolidone (NMP), dimethyl sulfoxide (DMSO), γ-butyrolactone (GBL), acetonitrile, acetone, *N,N*'-dimethylpropylene urea (DMPU), hexamethylphosphoric triamide (HMPA), dichloromethane, and chloroform are preferred. By using said polar solvents such as *N,N-*dimethylformamide (DMF), *N,N-*dimethylacetamide (DMAC), *N*-methylpyrrolidone (NMP), dimethyl sulfoxide (DMSO), γ-butyrolactone (GBL), acetonitrile, acetone, *N,N*'-dimethylpropylene urea (DMPU), hexamethylphosphoric triamide (HMPA), dichloromethane, and chloroform, a preferred reactivity may be ensured. γ-Butyrolactone (GBL) and acetone are more preferred. By using said γ-butyrolactone (GBL) and acetone, a more preferred reactivity may be ensured. Acetone is even more preferred. By using said acetone, an even more preferred reactivity may be ensured.

The solvent may be used alone or in combination thereof, if necessary. The solvent may be a commercially available one.

When the solvent is used, the amount of the solvent used, per mol of 5-(2-butyl-1,3-dioxolan-2-yl)-1-methylpentyl acetate (1), is preferably more than 0 up to 7,000 g, more preferably 50 to 3,000 g, and even more preferably 100 to 1,500 g. By using said preferred amount, said more preferred amount, and said even more preferred amount, a preferred reactivity, a more preferred reactivity, and an even more preferred reactivity may be ensured.

The reaction temperature of the deprotection varies, depending on the acid and/or the solvent to be used, and is preferably -15 to 180°C, more preferably 5 to 120°C, and even more preferably 40 to 80°C. By using said preferred reaction temperature, said more preferred reaction temperature, and said even more preferred reaction temperature, a preferred reactivity, a more preferred reactivity, and an even more preferred reactivity may be ensured.

The reaction time of the deprotection varies, depending on the acid and the solvent to be used and/or the production scale, and is preferably 0.5 to 100 hours.

### III. Process for preparing the aforesaid 10-acetoxy-5-undecanol (6)

(i) The process for preparing the 10-acetoxy-5-undecanol (6) will be explained in detail below.

10-Acetoxy-5-undecanol (6) may be prepared by reducing 10-acetoxy-5-undecanone (5) with a reducing agent.

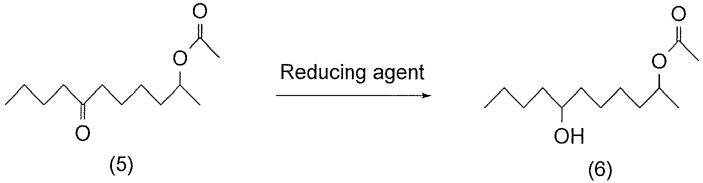

Examples of the reducing agent include alkali metal borohydrides such as lithium borohydride, sodium borohydride, potassium borohydride, and sodium triacetoxyborohydride; alkaline earth metal borohydrides such as magnesium borohydride and calcium borohydride; alkali metal cyanoborohydrides such as lithium cyanoborohydride, sodium cyanoborohydride, and potassium cyanoborohydride; alkaline earth metal cyanoborohydrides such as magnesium cyanoborohydride and calcium cyanoborohydride; alkali metal tri-sec-butyl borohydrides such as sodium tri-sec-butyl borohydride and lithium tri-sec-butyl borohydride; and diisobutylaluminum hydride, sodium bis(2-methoxyethoxy)aluminum hydride (Red-Al), and lithium aluminum hydride. Sodium borohydride, lithium borohydride, sodium triacetoxyborohydride, lithium cyanoborohydride, sodium cyanoborohydride, and potassium cyanoborohydride are preferred. By using said sodium borohydride, lithium borohydride, sodium triacetoxyborohydride, lithium cyanoborohydride, sodium cyanoborohydride, and potassium cyanoborohydride, a preferred selective reduction may be ensured. Sodium borohydride, sodium triacetoxyborohydride, and sodium cyanoborohydride are more preferred. By using said sodium borohydride, sodium triacetoxyborohydride, and sodium cyanoborohydride, a more preferred selective reduction may be ensured. Sodium borohydride is even more preferred. By using said sodium borohydride, an even more preferred selective reduction may be ensured.

The reducing agent may be used alone or in combination thereof, if necessary. A commercially available reducing agent may be used.

The amount of the reducing agent used varies, depending on the reducing agent to be used, and is preferably 0.25 to 5.0 mol, per mol of 10-acetoxy-5-undecanone (5). By using said preferred amount, a preferred reactivity may be ensured. When sodium borohydride is used as the reducing agent, the amount of the reducing agent used, per mol of 10-acetoxy-5-undecanone (5), is preferably 0.25 to 5.0 mol, more preferably 0.35 to 3.0 mol, and even more preferably 0.5 to 1.5 mol. By using said preferred amount, said more preferred amount, and said even more preferred amount, a preferred reactivity, a more preferred reactivity, and an even more preferred reactivity may be ensured.

A solvent may be incorporated in the reduction reaction, if necessary. Examples of the solvent include ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; polar solvents such as *N,N-*dimethylformamide (DMF), *N,N*-dimethylacetamide (DMAC), *N*-methylpyrrolidone (NMP), dimethyl sulfoxide (DMSO), γ-butyrolactone (GBL), acetonitrile, acetone, *N,N'-*dimethylpropylene urea (DMPU), hexamethylphosphoric triamide (HMPA), dichloromethane, and chloroform; ester solvents such as methyl acetate, ethyl acetate, n-propyl acetate, and n-butyl acetate; alcoholic solvents such as methanol and ethanol; and water.

An appropriate solvent may be selected according to the reducing agent used. When, for example, an alkali metal borohydride is used as the reducing agent, alcoholic solvents such as ethanol and mixed solvents of an alcoholic solvent and another solvent are preferred.

The solvent may be used alone or in combination thereof, if necessary. The solvent may be a commercially available one.

When the solvent is used, the amount of the solvent used, per mol of 10-acetoxy-5-undecanone (5), is preferably more than 0 up to 7,000 g, more preferably 50 to 3,000 g, and even more preferably 100 to 1,500 g. By using said preferred amount, said more preferred amount, and said even more preferred amount, a preferred reactivity, a more preferred reactivity, and an even more preferred reactivity may be ensured.

The solvent may be used alone or in combination thereof, if necessary. A commercially available solvent may be used.

A base may be incorporated in the reduction reaction, if necessary. Examples of the base include alkali metal hydroxides such as lithium hydroxide, sodium hydroxide, and potassium hydroxide; alkaline earth metal hydroxides such as calcium hydroxide, magnesium hydroxide, and barium hydroxide; alkali metal carbonates such as sodium carbonate and potassium carbonate; alkaline earth metal carbonates such as calcium carbonate, magnesium carbonate, and barium carbonate; alkali metal bicarbonates such as sodium bicarbonate and potassium bicarbonate; and alkaline earth metal bicarbonates such as calcium bicarbonate and magnesium bicarbonate. When, for example, an alkali metal borohydride is used as the reducing agent, the base is preferably alkali metal hydroxides such as sodium hydroxide. By using said alkali metal hydroxides such as sodium hydroxide, a preferred handling may be ensured. Sodium hydroxide and potassium hydroxide are more preferred. By using said sodium hydroxide and potassium hydroxide, a more preferred handling may be ensured. Sodium hydroxide is even more preferred. By using said sodium hydroxide, an even more preferred handling may be ensured.

The base may be used alone or in combination thereof, if necessary. A commercially available base may be used.

When the base is in a solid form, the base may be added as such to the reaction mixture, or may be dissolved in the solvent used in the reduction reaction.

The amount of the base used, per mol of 10-acetoxy-5-undecanone (5), is preferably more than 0 up to 10.00 mol, more preferably 0.0001 to 1.0 mol, and even more preferably 0.001 to 0.1 mol. By using said preferred amount, said more preferred amount, and said even more preferred amount, a preferred reactivity, a more preferred reactivity, and an even more preferred reactivity may be ensured.

The reaction temperature of the reduction reaction varies, depending on the reducing agent to be used, and is preferably -15 to 180°C, more preferably 0 to 100°C, and even more preferably 10 to 60°C. By using said preferred reaction temperature, said more preferred reaction temperature, and said even more preferred reaction temperature, a preferred reactivity, a more preferred reactivity, and an even more preferred reactivity may be ensured.

The reaction time of the reduction reaction varies, depending on the reducing agent and the solvent to be used and/or the production scale, and is preferably 0.5 to 100 hours.

10-Acetoxy-5-undecanol (6) is more fat-soluble than diols such as 2,7-undecanediol because the hydroxy group at position-10 is protected by an acetyl group. Recovery with high yield is therefore possible without aqueous layer loss, even without using a large amount of an extraction solvent.

### IV. 2,7-Diacetoxyundecane (7) and process for preparing 2,7-diacetoxyundecane (7)

(i) The process for preparing 2,7-diacetoxyundecane (7) will be described in detail below.

2,7-Diacetoxyundecane (7) may be prepared by acetylating the aforesaid 10-acetoxy-5-undecanol (6).

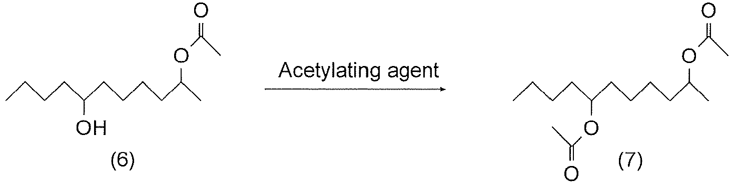

The acetylation reaction may be carried out, for example, by acetylating the hydroxy group with an acetylating agent.

Examples of the acetylating agent include acetic acid; acid anhydrides such as acetic anhydride; acetyl halide compounds such as acetyl chloride, acetyl bromide, and acetyl iodide; and acetate compounds such as methyl acetate, ethyl acetate, propyl acetate, butyl acetate, pentyl acetate, and hexyl acetate.

The acetylating agent is preferably acid anhydrides and acetyl halide compounds. By using said acid anhydrides and acetyl halide compounds, a preferred reactivity may be ensured. The acetylating agent is more preferably acid anhydrides. By using said acid anhydrides, a more preferred reactivity may be ensured. The acetylating agent is even more preferably acetic anhydride. By using said acetic anhydride, an even more preferred reactivity may be ensured.

The amount of the acetylating agent used, per mol of 10-acetoxy-5-undecanol (6), is preferably 0.8 to 15.0 mol, more preferably 1.0 to 10.0 mol, and even more preferably 1.2 to 3.0 mol. By using said preferred amount, said more preferred amount, and said even more preferred amount, a preferred reactivity and economy, a more preferred reactivity and economy, and an even more preferred reactivity and economy may be ensured.

An acid or a base may be incorporated in the acetylation, if necessary.

Examples of the acid include mineral acids such as hydrochloric acid, sulfuric acid, and nitric acid; aromatic sulfonic acids such as benzenesulfonic acid and p-toluenesulfonic acid; and Lewis acids such as aluminum trichloride, aluminum ethoxide, aluminum isopropoxide, aluminum oxide, boron trifluoride, boron trichloride, boron tribromide, magnesium chloride, magnesium bromide, magnesium iodide, zinc chloride, zinc bromide, zinc iodide, tin tetrachloride, tin tetrabromide, dibutyltin dichloride, dibutyltin dimethoxide, dibutyltin oxide, magnesium chloride, magnesium bromide, titanium tetrachloride, titanium tetrabromide, titanium(IV) methoxide, titanium(IV) ethoxide, titanium(IV) isopropoxide, and titanium(IV) oxide.

The acid may be used alone or in combination thereof, if necessary.

When the acid is used, the amount of the acid used, per mol of 10-acetoxy-5-undecanol (6), is preferably more than 0 up to 3.0 mol, more preferably 0.001 to 1.5 mol, and even more preferably 0.01 to 1.0 mol. By using said preferred amount, said more preferred amount, and said even more preferred amount, a preferred reactivity and economy, a more preferred reactivity and economy, and an even more preferred reactivity and economy may be ensured.

Examples of the base include the following compounds:
trialkylamine compounds such as trimethylamine, triethylamine, and *N,N-*diisopropylethylamine;
cyclic amine compounds such as piperidine, pyrrolidine, and 1,8-diazabicyclo[5.4.0]-7-undecene (DBU);
aromatic amine compounds such as pyridine, lutidine, N,N-dimethylaniline, N,N-diethylaniline, N,N-dibutylaniline, and 4-dimethylaminopyridine;
metal alkoxides such as sodium methoxide, sodium ethoxide, sodium t-butoxide, sodium t-amyloxide, lithium methoxide, lithium ethoxide, lithium t-butoxide, lithium t-amyloxide, potassium methoxide, potassium ethoxide, potassium t-butoxide, and potassium t-amyloxide;
alkyllithiums such as methyllithium, ethyllithium, propyllithium, n-butyllithium, sec-butyllithium, tert-butyllithium, pentyllithium, hexyllithium, heptyllithium, octyllithium, nonyllithium, and decyllithium;
Grignard reagents such as methylmagnesium chloride, methylmagnesium bromide, methylmagnesium iodide, ethylmagnesium chloride, ethylmagnesium bromide, ethylmagnesium iodide, propylmagnesium chloride, propylmagnesium bromide, propylmagnesium iodide, butylmagnesium chloride, butylmagnesium bromide, butylmagnesium iodide, pentylmagnesium chloride, pentylmagnesium bromide, pentylmagnesium iodide, hexylmagnesium chloride, hexylmagnesium bromide, hexylmagnesium iodide, heptylmagnesium chloride, heptylmagnesium bromide, heptylmagnesium iodide, octylmagnesium chloride, octylmagnesium bromide, octylmagnesium iodide, nonylmagnesium chloride, nonylmagnesium bromide, nonylmagnesium iodide, decylmagnesium chloride, decylmagnesium bromide, and decylmagnesium iodide;
metal acetylides such as lithium acetylide, sodium acetylide, potassium acetylide, calcium acetylide, and silver acetylide; and
inorganic metal salts such as potassium carbonate, sodium carbonate, and calcium carbonate.

The base may be used alone or in combination thereof, if necessary.

When the base is used, the amount of the base used, per mol of 10-acetoxy-5-undecanol (6), is preferably more than 0 up to 10.0 mol, more preferably 0.01 to 5.0 mol, and even more preferably 0.1 to 3.0 mol. By using said preferred amount, said more preferred amount, and said even more preferred amount, a preferred reactivity and economy, a more preferred reactivity and economy, and an even more preferred reactivity and economy may be ensured.

A solvent may be incorporated in the acetylation, if necessary.

Examples of the solvent include ether solvents such as tetrahydrofuran, 2-methyltetrahydrofuran (2-MeTHF), diethyl ether, dibutyl ether, 4-methyltetrahydropyran, cyclopentylmethylether, and 1,4-dioxane; hydrocarbon solvents such as hexane, heptane, benzene, toluene, xylene, and cumene; and polar solvents such as *N,N-*dimethylformamide, N,N-dimethylacetamide, N-methylpyrrolidone, dimethyl sulfoxide, γ-butyrolactone, acetonitrile, dichloromethane, chloroform, and hexamethylphosphoric triamide (HMPA). Ether solvents such as tetrahydrofuran, 2-methyltetrahydrofuran, and 4-methyltetrahydropyran; and hydrocarbon solvents such as toluene and xylene are preferred. By using said ether solvents such as tetrahydrofuran, 2-methyltetrahydrofuran, and 4-methyltetrahydropyran; and hydrocarbon solvents such as toluene and xylene, a preferred reactivity may be ensured.

The solvent may be used alone or in combination thereof, if necessary. The solvent may be a commercially available one.

A solvent may be incorporated in the acetylation, if necessary, or a solvent may not be used.

When the solvent is used, the amount of the solvent used in the acetylation, per mol of the aforesaid 10-acetoxy-5-undecanol (6), is preferably more than 0 up to 5,000 g, more preferably 30 to 2,000 g, and even more preferably 70 to 1,000 g.

The reaction temperature of the acetylation varies, depending on the acetylating agent and/or the solvent to be used, and is preferably -40 to 120°C, more preferably -20 to 100°C, and even more preferably 0 to 80°C. By using said preferred reaction temperature, said more preferred reaction temperature, and said even more preferred reaction temperature, a preferred reactivity, a more preferred reactivity, and an even more preferred reactivity may be ensured.

The reaction time of the acetylation is preferably 0.5 to 100 hours. By using said preferred reaction time, a preferred reactivity may be ensured.

Thus, according to the present invention, the synthetic intermediate, 5-(2-butyl-1,3-dioxolan-2-yl)-1-methylpentyl acetate (1), may be easily and efficiently prepared. According to the present invention, the sex pheromone of the Pear gall midge (scientific name: *Contarinia pyrivora*), 2,7-diacetoxyundecane (7), may be prepared efficiently from 5-(2-butyl-1,3-dioxolan-2-yl)-1-methylpentyl acetate (1) with fewer steps and with high ease of preparation. Specifically, 2,7-diacetoxyundecane (7) may be prepared efficiently because the target compound can be selectively synthesized without the need for separation by column chromatography or the like.

### EXAMPLES

The present invention will be described with reference to the following Examples. It should be noted that the present invention is not limited to or by the Examples.

The term "purity" as used herein means an area percentage in gas chromatography (GC), unless otherwise specified. The term "product ratio" means a ratio of area percentages in GC. The term "yield" is calculated from the area percentages determined by GC.

In the Examples, monitoring of the reactions and calculation of the yields were carried out in the following GC conditions.

GC conditions: GC: Capillary gas chromatograph GC-2014 (Shimadzu Corporation); column: DB-5, 0.25 µm x 0.25 mmϕ x 30 m; carrier gas: He (1.55 mL/min), detector: FID; column temperature: 150°C, elevated in a rate of 5°C/min, and up to 230°C.

The yield was calculated according to the following equation in consideration of purities (% GC) of a starting material and a product. Yield (%) = {[(weight of a product obtained by a reaction × % GC) / molecular weight of a product] ÷ [(weight of a starting material in a reaction × % GC) / molecular weight of a starting material] × 100

THF represents tetrahydrofuran, OFE represents triethyl orthoformate, 2-MeTHF represents 2-methyltetrahydrofuran, *p*-TsOH represents p-toluenesulfonic acid, and Ac₂O represents acetic anhydride.

### Example 1: Preparation of 2-butyl-2-(3-chloropropyl)-1,3-dioxolane compound (8: X¹ = Cl)

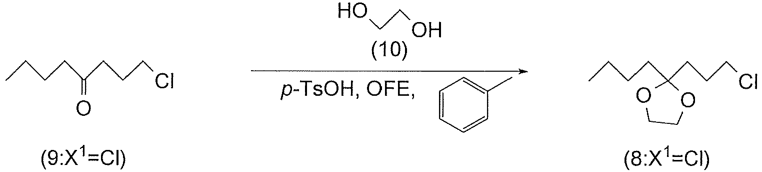

A 1-chloro-4-octanone (9: X¹ = Cl, 793.15 g, 4.78 mol, purity 98.07%), ethylene glycol (10) (326.50 g, 5.26 mol, purity 100%), *p*-toluenesulfonic acid monohydrate (1.65 g, 0.0087 mol, purity 100%), and toluene (1,653.81 g) were placed in a reactor at a room temperature and stirred for 32 minutes at 60 to 70°C. Trimethyl orthoformate (558.21 g, 5.26 mol, purity 100%) was then added dropwise to the reactor at 60 to 70°C. After the completion of the dropwise addition, the reaction mixture was stirred for 3.5 hours at 60 to 70°C. After cooling the internal temperature to 10 to 20°C, an aqueous solution (700.00 g) of 25% by mass sodium hydroxide was then added to the reaction mixture, followed by phase separation. An aqueous solution (10.00 g) of 25% by mass sodium hydroxide and water (100.00 g) were added and mixed with the organic layer, followed by phase separation. The organic layer thus obtained was concentrated at a reduced pressure, and the resulting concentrate was distilled at a reduced pressure to obtain a 2-butyl-2-(3-chloropropyl)-1,3-dioxolane compound (8: X¹ = Cl, 940.84 g, 4.38 mol, purity 96.32%, b.p. = 110.5 to 113.7°C/0.044 kPa (0.33 mmHg)) with a yield of 91.69%.

The following is the spectrum data of the 2-butyl-2-(3-chloropropyl)-1,3-dioxolane compound (8: X¹ = Cl) thus obtained.

Nuclear magnetic resonance spectrum: ¹H-NMR (500 MHz, CDCl₃): δ = 0.89 (3H, t, J = 7.3 Hz), 1.26-1.37 (4H, m), 1.52-1.61 (2H, m), 1.72-1.77 (2H, m), 1.81-1.88 (2H, m), 3.55 (2H, t, J = 6.9 Hz), 3.91-3.94 (4H, m); ¹³C-NMR (125 MHz, CDCl₃): δ = 14.01, 22.94, 25.95, 27.15, 34.27, 36.99, 45.30, 64.91, 111.30.

Mass spectrum: EI-mass spectrum (70 eV): m/z 207 (M⁺+1), 149, 129, 105, 77, 57.

Infrared absorption spectrum: (D-ATR): v = 2957, 2874, 1461, 1445, 1314, 1083, 1045, 948, 887, 652.

### Example 2: Preparation of [3-(2-butyl-1,3-dioxolan-2-yl)propyl]magnesium chloride (2: X¹ = Cl)

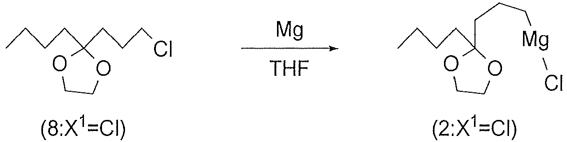

Magnesium (35.72 g, 1.47 gram atoms) and tetrahydrofuran (770.00 g) were placed in a reactor at a room temperature and stirred for 38 minutes at 55 to 60°C. The 2-butyl-2-(3-chloropropyl)-1,3-dioxolane compound (8: X¹ = Cl, 300.45 g, 1.40 mol, purity 96.32%) prepared in Example 1 was then added dropwise to the reactor at an internal temperature of 55 to 60°C. After the completion of the dropwise addition, the reaction mixture was stirred for 2 hours at 50 to 60°C to obtain [3-(2-butyl-1,3-dioxolan-2-yl)propyl]magnesium chloride (2: X¹ = Cl).

The following is the spectrum data of [3-(2-butyl-1,3-dioxolan-2-yl)propyl]magnesium chloride (2: X¹ = Cl) thus obtained.

Nuclear magnetic resonance spectrum: ¹H-NMR (500 MHz, CDCl₃): δ = -0.72 (2H, t, J = 7.2 Hz), 0.83 (3H, t, J = 7.2 Hz), 1.19-1.38 (4H, m), 1.40-1.63 (6H, m), 3.75-3.88 (4H, m); ¹³C-NMR (125 MHz, CDCl₃): δ = 7.76, 14.24, 23.66, 24.29, 26.80, 36.21, 44.29, 64.73, 67.21, 113.76.

Infrared absorption spectrum: (D-ATR): v = 1365, 1033, 950, 516.

### Example 3: Preparation of 5-(2-butyl-1,3-dioxolan-2-yl)-1-methylpentyl acetate (1)

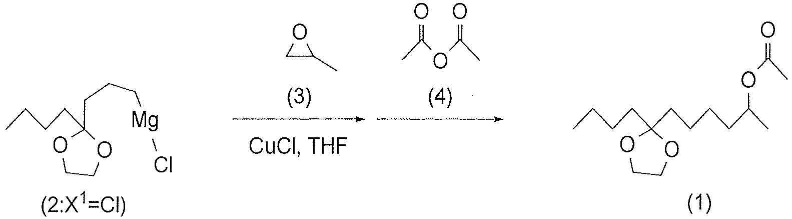

Next, cuprous chloride (CuCl) (0.29 g, 0.0027 mol, purity 95%) was added to the reactor containing [3-(2-butyl-1,3-dioxolan-2-yl)propyl]magnesium chloride (2: X¹ = Cl) prepared in Example 2, and the reaction mixture was stirred for 19 minutes at an internal temperature of 0 to 10°C. Propylene oxide (3) (93.51 g, 1.61 mol, purity 100%) was then added dropwise at 0 to 20°C. After the completion of the dropwise addition, the reaction mixture was stirred for 2.5 hours at 15 to 25°C. Acetic anhydride (4) (164.36 g, 1.61 mol, purity 100%) was then added dropwise to the reaction mixture at 20 to 60°C. After the completion of the dropwise addition, the reaction mixture was stirred for 1.5 hours at 30 to 35°C. An aqueous solution of acetic acid (prepared from acetic acid (164.36 g) and water (541.33 g)) was then added to the reaction mixture, followed by phase separation. The organic layer thus obtained was concentrated at a reduced pressure, and the resulting concentrate was distilled at a reduced pressure to obtain 5-(2-butyl-1,3-dioxolan-2-yl)-1-methylpentyl acetate (1) (347.47 g, 1.17 mol, purity 91.90%, b.p. = 115.8 to 138.5°C/0.056 kPa (0.42 mmHg)) with a yield of 83.75%.

The following is the spectrum data of 5-(2-butyl-1,3-dioxolan-2-yl)-1-methylpentyl acetate (1) thus obtained.

Nuclear magnetic resonance spectrum: ¹H-NMR (500 MHz, CDCl₃): δ = 0.88 (3H, t, J = 6.9 Hz), 1.18 (3H, d, J = 6.5 Hz), 1.23-1.39 (8H, m), 1.41-1.61 (7H, m), 2.00 (3H, s), 3.90 (3H, s), 4.86 (1H, tq, J = 6.2 Hz, 6.2 Hz); ¹³C-NMR (125 MHz, CDCl₃): δ = 14.01, 19.88, 21.32,22.96, 23.64, 25.66, 25.99, 35.86, 36.84, 36.98, 64.86, 70.96, 111.70, 170.71.

Mass spectrum: EI-mass spectrum (70 eV): m/z 241 (M⁺-31), 215, 155, 129, 99, 57, 43.

Infrared absorption spectrum: (D-ATR): v = 2945, 2872, 1737, 1465, 1372, 1245, 1079, 1024, 949.

### Example 4: Preparation of 10-acetoxy-5-undecanone (5)

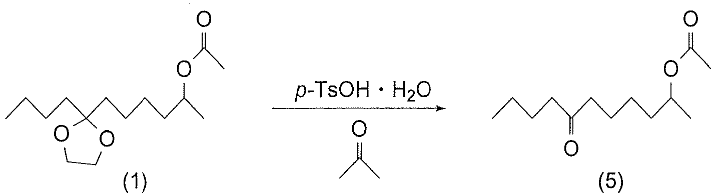

5-(2-Butyl-1,3-dioxolan-2-yl)-1-methylpentyl acetate (1) (334.20 g, 1.13 mol, purity 91.90%) prepared in Example 3, water (40.63 g, 2.26 mol), acetone (600.00 g), and p-toluenesulfonic acid monohydrate (2.15 g, 0.011 mol) were placed in a reactor at a room temperature and stirred for 2.5 hours at 60 to 65°C. After cooling the internal temperature to 10 to 20°C, an aqueous solution (3.24 g) of 25% by mass sodium hydroxide was then added to the reaction mixture. The reaction mixture was concentrated at a reduced pressure, and acetone was removed. The organic layer thus obtained was washed with sodium bicarbonate (3.12 g) and water (156.00 g), followed by phase separation. The organic layer thus obtained was concentrated again at a reduced pressure, and the resulting concentrate was distilled at a reduced pressure to obtain 10-acetoxy-5-undecanone (5) (269.86 g, 1.10 mol, purity 92.65%, b.p. = 120.0 to 129.0°C/0.39 kPa (2.9 mmHg)) with a yield of 97.04%.

The following is the spectrum data of 10-acetoxy-5-undecanone (5) thus obtained.

Nuclear magnetic resonance spectrum: ¹H-NMR (500 MHz, CDCl₃): δ =; 0.88 (3H, t, J = 7.3 Hz), 1.17 (3H, d, J = 6.1 Hz), 1.28 (4H, sext-like, J = 7.3 Hz), 1.40-1.61 (6H, m), 2.00 (3H, s), 2.37 (4H, q-like, J = 7.3 Hz), 4.86 (1H, tq, J = 6.1 Hz, 6.1 Hz)¹³C-NMR (125 MHz, CDCl₃): δ = 13.79, 19.88, 21.29, 22.31, 23.54, 25.01, 25.92, 35.67, 42.47, 42.50, 70.71, 170.68, 211.09.

Mass spectrum: EI-mass spectrum (70 eV): m/z 228 (M⁺), 186, 168, 153, 139, 126, 111, 97, 85, 69, 57, 43.

Infrared absorption spectrum: (D-ATR): v = 2936, 2872, 1735, 1715, 1463, 1372, 1245, 1131, 1023.

### Example 5: Preparation of 10-acetoxy-5-undecanol (6)

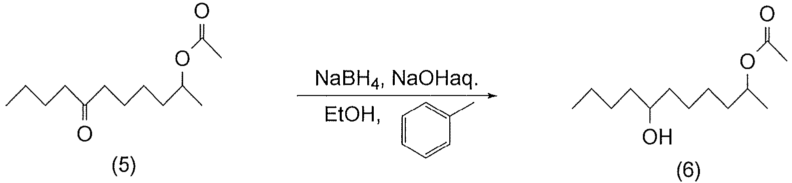

Sodium borohydride (NaBH₄) (24.49 g, 0.65 mol, purity 100%), ethanol (EtOH) (118.26 g), an aqueous solution (4.08 g) of 25% by mass sodium hydroxide (NaOHaq) and water (95.17 g) were placed in a reactor at a room temperature and stirred for 4 minutes at 20 to 25°C. A mixture of 10-acetoxy-5-undecanone (5) (265.96 g, 1.08 mol, purity 92.65%) prepared in Example 4 and toluene (118.26 g) was then added dropwise to the reactor at 25 to 40°C. After the completion of the dropwise addition, the reaction mixture was stirred for 3 hours at 30 to 40°C. The aqueous layer was then separated from the reaction mixture, and an aqueous solution of acetic acid (prepared from acetic acid (12.08 g) and water (120.77 g)) was added and mixed with the organic layer thus obtained, followed by phase separation. The organic layer thus obtained was concentrated at a reduced pressure, and the resulting concentrate was distilled at a reduced pressure to obtain, as a product, 10-acetoxy-5-undecanol (6) (256.54 g, 1.01 mol, purity 90.87%, b.p. = 121.0 to 132.9°C/0.24 kPa (1.8 mmHg)) with a yield of 93.80%. The product thus obtained included several percent of 2,7-diacetoxyundecane (7) together with 10-acetoxy-5-undecanol (6).

The following is the spectrum data of 10-acetoxy-5-undecanol (6) thus obtained.

Nuclear magnetic resonance spectrum: ¹H-NMR (500 MHz, CDCl₃): δ = 0.89 (3H, t, J = 7.3 Hz), 1.19 (3H, d, J = 6.1 Hz), 1.23-1.63 (14H, m), 1.61 (1H, br.s), 2.01 (3H, s), 3.52-3.62 (1H, m), 4.84-4.92 (1H, m); ¹³C-NMR (125 MHz, CDCl₃): δ = 14.02, 19.90, 19.93, 21.32, 22.71, 25.40, 25.44, 27.79, 35.85, 35.88, 37.17, 37.25, 70.89, 70.92, 71.72, 71.78, 170.79.

Mass spectrum: EI-mass spectrum (70 eV): m/z 213 (M⁺-17), 129, 113, 95, 69, 56, 43.

Infrared absorption spectrum: (D-ATR): v = 3420, 2933, 2860, 1738, 1717, 1464, 1373, 1245, 1127, 1023.

### Example 6: Preparation of 2,7-diacetoxyundecane (7)

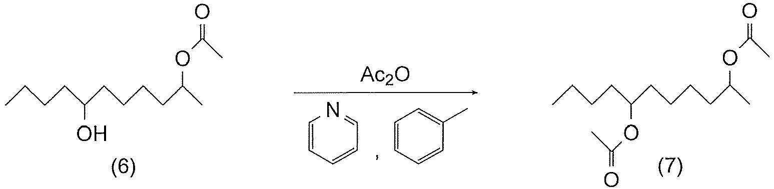

10-Acetoxy-5-undecanol (6) (253.64 g, 1.00 mol, purity 90.87%) prepared in Example 5, pyridine (166.28 g, 2.10 mol, purity 100%), and toluene (109.71 g) were placed in a reactor at a room temperature and stirred for 2 minutes at 20 to 30°C. Acetic anhydride (Ac₂O) (183.95 g, 1.80 mol, purity 100%) was then added dropwise to the reactor at 25 to 35°C. After the completion of the dropwise addition, the reaction mixture was stirred for 19 hours at 30 to 35°C. Water (268.89 g) was then added to the reaction mixture, followed by phase separation. Sodium chloride (8.07 g), sodium bicarbonate (10.13 g), and water (101.12 g) were added and mixed with the organic layer thus obtained, followed by phase separation. The organic layer thus obtained was then concentrated at a reduced pressure, and the resulting concentrate was distilled at a reduced pressure to obtain 2,7-diacetoxyundecane (7) (301.84 g, 1.07 mol, purity 96.65%, b.p. = 126.1 to 132.1°C/0.043 kPa (0.32 mmHg)) with a yield of 107.00%. The starting material of Example 5 included 2,7-diacetoxyundecane (7) together with 10-acetoxy-5-undecanol (6), and so the yield calculated for the two steps of Examples 5 and 6 was 99.26%.

The following is the spectrum data of 2,7-diacetoxyundecane (7) thus obtained.

Nuclear magnetic resonance spectrum: ¹H-NMR (500 MHz, CDCl₃): δ = 0.87 (3H, t, J = 7.3 Hz), 1.18 (3H, d, J = 6.1 Hz), 1.20-1.39 (8H, m), 1.39-1.60 (6H, m), 2.00 (3H, s), 2.02 (3H, d, J = 0.8 Hz), 4.80-4.90 (2H, m); ¹³C-NMR (125 MHz, CDCl₃): δ = 13.92, 19.88, 19.91, 21.20, 21.30, 22.54, 25.11, 25.25, 27.43, 33.76, 33.78, 33.96, 35.75, 35.77, 70.80, 74.13, 74.16, 170.69, 170.85.

Mass spectrum: EI-mass spectrum (70 eV): m/z 273 (M⁺+1), 129, 113, 95, 68, 43.

Infrared absorption spectrum: (D-ATR): v = 2936, 2862, 1737, 1373, 1243, 1022.

### Reference Example 1: Preparation of 2-(1-butylcyclobutan-1-yloxy)ethanol (11)

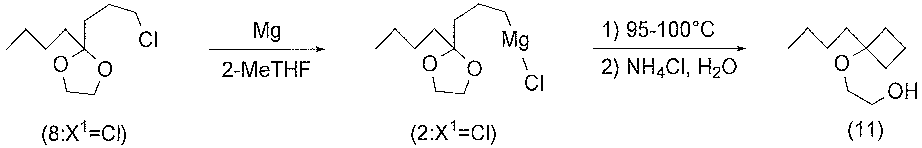

Magnesium (Mg) (1.07 g, 0.044 gram atoms) and 2-methyltetrahydrofuran (12.00 g) were placed in a reactor at a room temperature and stirred for 22 minutes at 60 to 65°C. The 2-butyl-2-(3-chloropropyl)-1,3-dioxolane compound (8: X¹ = Cl, 8.58 g, 0.040 mol, purity 96.32%) prepared in Example 1 was then added dropwise to the reactor at 60 to 65°C. After the completion of the dropwise addition, the reaction mixture was stirred for 3 hours at 75 to 80°C, and then stirred for 3 hours at 95 to 100°C. An aqueous solution of ammonium chloride (prepared from ammonium chloride (NH₄Cl) (1.00 g) and water (40.00 g)) and 20% by mass hydrochloric acid (10.00 g) were then added to the reaction mixture, followed by phase separation. The organic layer thus obtained was then concentrated at a reduced pressure, and the resulting concentrate was distilled at a reduced pressure to obtain 2-(1-butylcyclobutan-1-yloxy)ethanol (11) (5.26 g, 0.028 mol, purity 91.81%, b.p. = 85.0 to 96.0°C/0.45 kPa (3.4 mmHg)) with a yield of 70.09%.

The following is the spectrum data of 2-(1-butylcyclobutan-1-yloxy)ethanol (11) thus obtained.

Nuclear magnetic resonance spectrum: ¹H-NMR (500 MHz, CDCl₃): δ = 0.91 (3H, t, J = 7.3 Hz), 1.23-1.37 (4H, m), 1.51-1.63 (3H, m), 1.69-1.78 (1H, m), 1.83-1.90 (2H, m), 2.05 (2H, dq, J = 2.7 Hz, 9.6 Hz), 2.22 (1H, br.s), 3.33 (2H, t, J = 4.6 Hz), 3.69 (2H, t, J = 4.6 Hz); ¹³C-NMR (125 MHz, CDCl₃): δ = 12.58, 14.09, 23.01, 25.05, 32.01, 34.93, 62.10, 62.47, 79.29.

Mass spectrum: EI-mass spectrum (70 eV): m/z 172 (M⁺), 157, 143, 129, 115, 102, 83, 71, 59, 41.

Infrared absorption spectrum: (D-ATR): v = 3423, 2957, 2932, 2862, 1459, 1256, 1054, 963, 891.

## Claims

1. 5-(2-Butyl-1,3-dioxolan-2-yl)-1-methylpentyl acetate of the following formula (1):

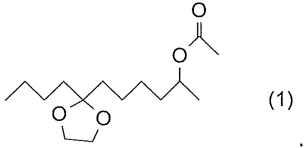

2. A process for preparing 5-(2-butyl-1,3-dioxolan-2-yl)-1-methylpentyl acetate of the following formula (1): 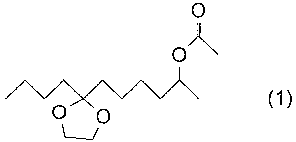 the process comprising the step of:
subjecting an organic magnesium compound (2) of the following general formula (2): 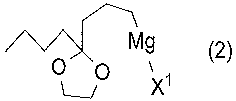 wherein X¹ represents a halogen atom or a 3-(2-butyl-1,3-dioxolan-2-yl)propyl group, to a nucleophilic addition reaction with propylene oxide of the following formula (3): 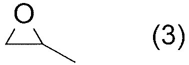 and then reacting with an acetylating agent of the following general formula (4): 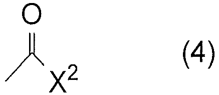 wherein X² represents a halogen atom, an acetoxy group, a methoxy group, or an ethoxy group,
to form 5-(2-butyl-1,3-dioxolan-2-yl)-1-methylpentyl acetate (1).

3. A process for preparing 10-acetoxy-5-undecanone of the following formula (5): 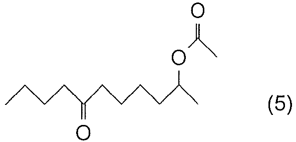 the process comprising the step of:
eliminating a ketal from 5-(2-butyl-1,3-dioxolan-2-yl)-1-methylpentyl acetate of the following formula (1): 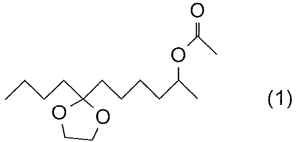 in the presence of an acid or a peroxide to form 10-acetoxy-5-undecanone (5).

4. A process for preparing 2,7-diacetoxyundecane of the following formula (7): 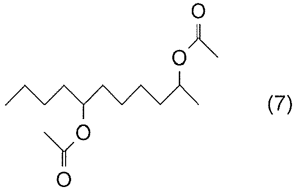 the process comprising the steps of:
preparing the aforesaid10-acetoxy-5-undecanone (5) according to claim 3,
subjecting the 10-acetoxy-5-undecanone (5) to a reduction reaction to form 10-acetoxy-5-undecanol of the following formula (6): 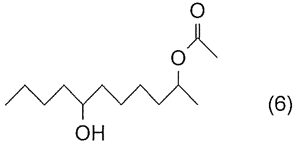 and
subjecting the 10-acetoxy-5-undecanol (6) to an acetylation reaction to form 2,7-diacetoxyundecane (7).
